# EUROPÄISCHE PATENTSCHRIFT

(11) **EP 1 968 952 B1**
(45) Veröffentlichungstag und Bekanntmachung des Hinweises auf die Patenterteilung: **10.08.2011**
(21) Anmeldenummer: 06847036.8
(22) Anmeldetag: 21.12.2006
(51) Int. Cl.: C07D 243/04

(54) **3-(4-PIPERIDINYL)-2,3,4,5-TETRAHYDRO-1,3-BENZODIAZEPIN-2(1H)-ON**
3-(4-PIPERIDINYL)-2,3,4,5-TETRAHYDRO-1,3-BENZODIAZEPINE-2(1H)-ONE
3-(4-PIPERIDINYL)-2,3,4,5-TETRAHYDRO-1,3-BENZODIAZEPINE-2(1H)-ONE

(30) Priorität: 24.12.2005 EP 05028476
(43) Veröffentlichungstag der Anmeldung: 17.09.2008
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: SCHNAUBELT, Juergen, 88447 Oberhoefen/Warthausen (DE); STEHLE, Emanuel, 88213 Ravensburg (DE); KRUEGER, Thomas, 88353 Kisslegg (DE)
(74) Vertreter: Hammann, Heinz
(86) Internationale Anmeldenummer: PCT/EP2006/070067
(87) Internationale Veröffentlichungsnummer: WO 2007/074130

(56) Entgegenhaltungen:
- EP-A- 1 619 187
- WO-A-2005/065779
- US-A1- 2004 204 397
- DATABASE BEILSTEIN Beilstein Institut zur Förderung der Wissenschaften, Frankfurt/Main, DE; XP002381997 & TETRAHEDRON, Bd. 55, Nr. 15, 1999, Seiten 4699-4708,
- DATABASE BEILSTEIN Beilstein Institut zur Förderung der Wissenschaften, Frankfurt/Main, DE; XP002381998 & INDIAN J. CHEM. SECT. B, Bd. 43, Nr. 7, 2004, Seiten 1537-1543,

## Beschreibung

Gegenstand der vorliegenden Erfindung ist ein Verfahren zur Herstellung der Verbindung 3-(4-Piperidinyl)-2,3,4,5-tetrahydro-1,3-benzodiazepin-2(1*H*)-on der Formel **I** 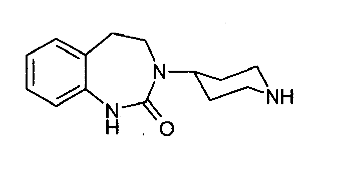 welche als Strukturelement in CGRP-Antagonisten, die sich vor allem zur oralen Therapie von Migräne eignen, zu finden ist.

### HINTERGRUND DER ERFINDUNG

Beispiele für Verbindungen mit CGRP-antagonistischen Eigenschaften, die als Strukturelement die Verbindung der Formel **I** enthalten, werden in den internationalen Patentanmeldungen PCT/EP97/04862, PCT/EP00/02004, PCT/EP00/13236, PCT/EP03/02417, PCT/EP03/11762 und PCT/EP03/11763 beschrieben. Verfahren zur Herstellung der Verbindung I sind aus WO 2005/065779 und 2004/204397 bekannt.

### KURZE ZUSAMMENFASSUNG DER ERFINDUNG

Gegenstand der vorliegenden Anmeldung ist ein Verfahren zur Herstellung der Verbindung 3-(4-Piperidinyl)-2,3,4,5-tetrahydro-1,3-benzodiazepin-2(1*H*)-on der Formel **I**. Das erfindungsgemäße Verfahren führt in wenigen Schritten zu guten Ausbeuten der gewünschten Verbindung, die zudem sehr rein anfällt.

### DETAILLIERTE BESCHREIBUNG DER ERFINDUNG

Als Ausgangsstoff für die Verbindung der Formel **I** kann 2-Nitrophenylessigsäure verwendet werden. Sie wird in einem ersten Schritt mit einer äquimolaren Lösung aus 4-Amino-*N*-phenylmethylpiperidin in Gegenwart von mindestens einem Äquivalent, vorzugsweise 1.1 bis 1.5 Äquivalenten, besonders bevorzugt 1.1 Äquivalenten, Kondensationsmittel wie Carbonyldiimidazol, Carbonylditriazol, n-Propanphosphonsäureanhydrid, Dicyclohexylcarbodiimid, Thionylchlorid, TBTU O-(Benzotriazol-1-yl)-*N,N,N',N'*-tetramethyluronium-tetrafluoroborat oder 1-Ethyl-3-(3'-dimethylaminopropyl)-carbodiimid zum 2-Nitro-*N*-[1-(phenylmethyl)-4-piperidinyl]-phenylacetamid der Formel **II** 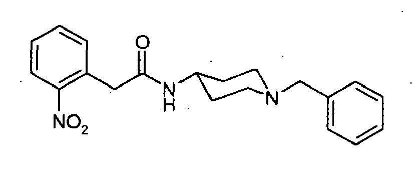 umgesetzt. Geeignete Lösungsmittel sind polare aprotische Lösungsmittel wie Tetrahydrofuran, 2-Methyltetrahydrofuran, Dimethoxyethan, Toluol, Dimethylformamid oder *N*-Methylpyrrolidinon.
Das Produkt kann z.B. durch Verdünnen mit Wasser zum Auskristallisieren gebracht und durch Filtration oder Zentrifugation und Trocknung aufgearbeitet werden.

Im folgenden Schritt wird das 2-Nitro-*N*-[1-(phenylmethyl)-4-piperidinyl]-phenylacetamid der Formel **II** zuerst in einem polaren organischen Lösungsmittel, wie beispielsweise Methanol, Ethanol, Isopropanol, Dimethylformamid, *N*-Methylpyrrolidinon, 2-Methyltetrahydrofuran, Tetrahydrofuran, Dioxan oder Dimethoxyethan, gelöst und die Nitrogruppe durch Zugabe eines Metallkatalysators, vorzugsweise 2.5 bis 20%, besonders bevorzugt 4 bis 6%, zu einer Verbindung der Formel **III** 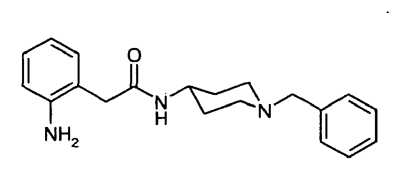 hydriert. Als Katalysatoren kommen Raney-Nickel oder Platindioxid in Frage. Vorteilhafte Bedingungen für die Hydrierung sind Temperaturen von 20 bis 60°C und ein Wasserstoffüberdruck von maximal 3 bar. Nach dem Abfiltrieren des Katalysators kann das Hydrierungsprodukt durch Abdestillieren des Lösungsmittels aufkonzentriert werden.

Anschließend erfolgt die Carbamoylierung des Rohproduktes der Formel **II** in Lösung durch Zugabe von mindestens einem Äquivalent, vorzugsweise 1.0 bis 2.0 Äquivalenten, besonders bevorzugt 1.6 Äquivalenten, eines Esters und in Gegenwart von mindestens einem Äquivalent, vorzugsweise 1.0 bis 2.0 Äquivalenten, besonders bevorzugt 1.6 Äquivalenten, einer Base zu einem Carbamat der allgemeinen Formel **IV** 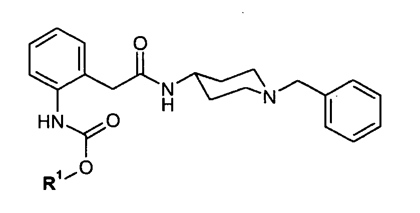 worin **R¹** eine Phenyl-, Benzyl-, eine Allyl-, 2,2,2-Trichlorethyl-, oder eine geradkettige oder verzweigte C₁₋₆-Alkylgruppe, beispielsweise die Methyl-, Ethyl-, Propyl-, *iso*-Propyl-, Butyl-, *iso*-Butyl-, *tert.*-Butyl-, Pentyl-, Neopentyl- oder Hexylgruppe, bedeutet. Als Ester können Pyrokohlensäure-di-*tert.*-butylester oder ein Chlorameisensäureester, beispielsweise Chlorameisensäureallylester, -methylester, -ethylester, -2,2,2-trichlorethylester, -propylester, -*iso*-propylester, -butylester, -isobutylester, -pentylester, -neopentylester, -hexylester, -phenylester oder -benzylester, verwendet werden. Als Base dient entweder ein aliphatisches Amin, wie beispielsweise *N*-Methylmorpholin, oder eine wässrige Lösung von Natriumcarbonat, Kaliumcarbonat, Natriumhydroxid, Ammoniak oder Kaliumhydroxid. Zu dem Reaktionsgemisch wird Wasser zugegeben und nach Phasentrennung wird die organische Phase im Vakuum aufkonzentriert.
Das Produkt kann beispielsweise durch Zugabe einer Aceton-Wasser-Mischung auskristallisiert werden. Das Produkt kann dann durch Filtration oder Zentrifugation und Trocknung aufgearbeitet werden.

Im folgenden Schlüsselschritt wird die Verbindung der allgemeinen Formel **IV** zuerst in einem aprotischen organischen Lösungsmittel, wie beispielsweise Toluol, 2-Methyltetrahydrofuran, Tetrahydrofuran oder Dimethoxyethan, gelöst und die Carbonylgruppe durch Zugabe von mindestens 2 Äquivalenten, vorzugsweise 4.0 bis 8.0 Äquivalenten, eines Reduktionsmittels in eine Methylengruppe übergeführt.
Als Reduktionsmittel kommen beispielsweise Boran, Diisobutylaluminiumhydrid, Lithiumaluminiumhydrid, Lithium- oder Natriumborhydrid in Frage, gegebenenfalls unter Zusatz von mindestens 0.5 Äquivalenten, vorzugsweise 2.0 bis 4.0 Äquivalenten, einer Lewissäure, einer Säure oder eines Halogens, beispielsweise unter Zusatz von Schwefelsäure, Chlortrimethylsilan oder lod. Die Reduktion kann bei Temperaturen von 20 bis 100°C, vorzugsweise bei 60 bis 80°C, durchgeführt werden.
Unter diesen Bedingungen erfolgt die Cyclisierung zu 3-[1-(Phenylmethyl)-4-piperidinyl]-2,3,4,5-tetrahydro-1,3-benzodiazepin-2(1*H*)-on der Formel **V** 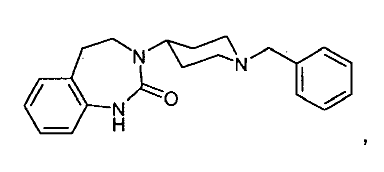 welches nach wässriger Aufarbeitung durch Verdünnen mit Alkohol, beispielsweise Methanol, Ethanol oder Isopropanol, vorzugsweise Methanol, ausgefällt und durch Filtration oder Zentrifugation und Trocknung aufgearbeitet werden kann.

In dem letzten Schritt wird die Benzylschutzgruppe des 3-[1-(Phenylmethyl)-4-piperidinyl]-2,3,4,5-tetrahydro-1,3-benzodiazepin-2(1*H*)-ons der Formel **V** abgespalten. Dazu wird der Ausgangsstoff in einem polaren Lösungsmittel, wie beispielsweise Methanol, Ethanol, Wasser, Aceton, Tetrahydrofuran, Dimethylformamid oder Propanol, gelöst, und in einem Druckreaktor hydriert. Als Hydrierungsmittel können beispielsweise Pd/C oder Pd(OH)₂ verwendet werden. Vorteilhafte Bedingungen für die Hydrierung sind Temperaturen von 40 bis 80°C und ein Wasserstoffüberdruck von maximal 3 bar. Nach dem Abfiltrieren des Katalysators kann das Hydrierungsprodukt 3-(4-Piperidinyl)-2,3,4,5-tetrahydro-1,3-benzodiazepin-2(1*H*)-on der Formel I durch Aufkonzentrierung des Lösungsmittels und anschließender Zugabe von Aceton oder Wasser kristallisiert, abfiltriert und getrocknet werden.

### EXPERIMENTELLER TEIL

### Beispiel 1: 2-Nitro-N-[1-(phenylmethyl)-4-piperidinyl]-phenylacetamid

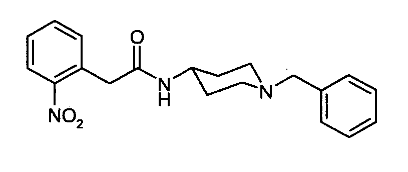

11.81 kg (72.87 mol, 1.1 eq) 1,1-Carbonyldiimidazol (CDI) werden bei 20°C vorgelegt und 18 L Tetrahydrofuran zugegeben. Anschließend werden 12.00 kg (66.24 mol, 1.0 eq) 2-Nitrophenylessigsäure, gelöst in 24 L Tetrahydrofuran, innerhalb von 15 Minuten zugegeben. Das Zulaufgefäß wird mit 9 L Tetrahydrofuran gespült und das Reaktionsgemisch 30 Minuten gerührt (Gasentwicklung: CO₂). Anschließend wird zweimal ein Vakuum von 300 mbar angelegt, um überschüssiges CO₂ zu entfernen.
Zur Lösung werden bei 20°C 12.61 kg (66.24 mol, 1.0 eq) 4-Amino-*N*-phenylmethylpiperidin in 6 L Tetrahydrofuran geben (exotherm). Nach der Zugabe wird das Reaktionsgemisch noch 2 Stunden bei 20°C gerührt. Danach werden 144 L Wasser zugegeben, wobei die Lösung nach Zugabe von ¼ der Wassermenge mit 2-Nitro-*N*-[1-(phenylmethyl)-4-piperidinyl]-phenylacetamid angeimpft wird. Die erhaltene Suspension wird auf 0 bis 5°C abgekühlt und zur Vervollständigung der Kristallisation eine weitere Stunde gerührt. Anschließend wird das Produkt abzentrifugiert, mit einem kalten Gemisch aus 160 L Wasser und 9 L Tetrahydrofuran gewaschen und unter Inertisierung im Trockenschrank bei 45°C getrocknet.
Ausbeute: 18.21 kg (77.8% der Theorie)
Chemische Reinheit laut HPLC: 99.8 %

### Beispiel 2: 2-Amino-N-[1-(phenylmethyl)-4-piperidinyl]-phenylacetamid

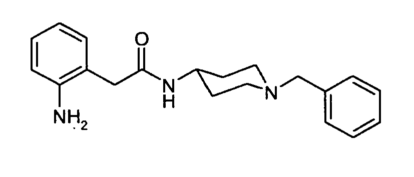

20.00 g (56.59 mmol, 1.0 eq) 2-Nitro-*N*-[1-(phenylmethyl)-4-piperidinyl]-phenylacetamid aus Beispiel 1 werden in 280 mL 2-Methyltetrahydrofuran vorgelegt. Die Lösung wird in Gegenwart von 2.00 g Platindioxid bei 20°C hydriert. Der Katalysator wird abfiltriert und mit 20 ml 2-Methyltetrahydrofuran gewaschen. Das Produkt wird nicht isoliert, sondern als Lösung in der nächsten Stufe eingesetzt.

### Beispiel 3: 2-Methoxycarbonylamino-N-[1-(phenylmethyl)-4-piperidinyl]-phenyl-acetamid

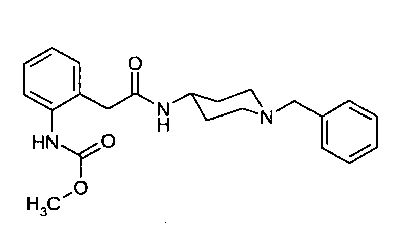

Aus der Lösung bestehend aus 9.15 g (28.29 mmol, 1.0 eq) 2-Amino-*N*-[1-(phenylmethyl)-4-piperidinyl]-phenylacetamid in 162 ml 2-Methyltetrahydrfuran werden 50 ml 2-Methyltetrahydrofuran abdestilliert. Die Lösung wird auf -12°C abgekühlt und es werden 4.56 g (45.10 mmol, 1.6 eq) 4-Methylmorpholin gefolgt von 4.26 g (45.10 mmol, 1.6 eq) Chlorameisensäuremethylester zugegeben Die Reaktionsmischung wird noch 1 Stunde bei 20°C gerührt und anschließend mit 100 ml Wasser versetzt. Nach Phasentrennung wir die Wasserphase mit 20 ml 2-Methyltetrahydrofuran gewaschen und die vereinten organischen Phasen mit 50 ml Wasser gewaschen. Das organische Lösungsmittel wird im Vakuum abdestilliert. Der Rückstand wird mit 50 ml Aceton versetzt und auf Rückfluss erhitzt. Anschließend werden 100 ml Wasser zugegeben und die erhaltene Suspension wird auf 20°C abgekühlt. Zur Vervollständigung der Kristallisation wird 30 Minuten bei 20°C gerührt. Anschließend wird das Produkt abgesaugt, mit 30 ml Aceton-Wasser-Mischung (1:2) gewaschen und unter Inertisierung im Tröckenschrank bei 40°C getrocknet.
Ausbeute: 8.68 g (80.0% der Theorie)
Chemische Reinheit laut HPLC: 96.8 %

### Beispiel 4: 3-[1-(Phenylmethyl)-4-piperidinyl]-2,3,4,5-tetrahydro-1,3-benzodiazepin-2(1H)-on

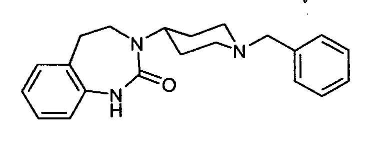

5.00 g (13.11 mmol, 1.0 eq) 2-Methoxycarbonylamino-*N*-[1-(phenylmethyl)-4-piperidinyl]-phenylacetamid aus Beispiel 3 werden in 50 ml Toluol suspendiert und auf 80°C aufgeheizt. Bei dieser Temperatur werden 56.1 g (78.61 mmol, 6.0 eq) Diisobutylaluminiumhydrid in Toluol (20%) zudosiert. Nach beendeter Zugabe wird das Reaktionsgemisch noch 30 Minuten bei dieser Temperatur gerührt. Nach Abkühlen auf 10°C wird mit Salzsäure auf pH = 2.5 eingestellt. Der entstandene Niederschlag wird mit Natronlauge aufgelöst und nach Phasentrennung wird die organische Phase mit 50 ml Wasser gewaschen. Die organische Phase wird im Vakuum bis zu einem Öl eingeengt und mit 50 ml Methanol versetzt.
Die erhaltene Lösung wird zum Rückfluss erhitzt und langsam auf -15 °C abgekühlt. Anschließend wird der Niederschlag abfiltriert und bei 50°C im Trockenschrank getrocknet.
Ausbeute: 1.86 g (42% der Theorie)

### Beispiel 5: 3-(4-Piperidiny)-2,3,4,5-tetrahydro-1,3-benzodiazepin-2(1H)-on

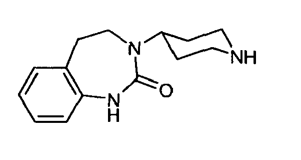

10.00 kg (29.81 mol, 1.0 eq) 3-[1-(Phenylmethyl)-4-piperidinyl]-2,3,4,5-tetrahydro-1,3-benzodiazepin-2(1*H*)-on aus Beispiel 3 werden in 100 L Methanol gelöst, mit 1.00 kg 10%-igem Pd/C versetzt und im Druckreaktor bei 70°C und 3 bar hydriert. Nach Beendigung der Wasserstoffaufnahme wird der Katalysator abfiltriert und mit 30 L Methanol gewaschen. Das Filtrat wird im Vakuum aufkonzentriert und der Rückstand in 100 L Aceton suspendiert. Danach wird zum Rückfluss erhitzt, die Suspension 15 Minuten unter Rückfluss gerührt und die Hälfte des Acetons bei Normaldruck abdestilliert. Nach beendeter Destillation wird auf 0°C abgekühlt und eine weitere Stunde gerührt. Das Produkt wird abgesaugt, mit 20 L Aceton gewaschen und bei 50°C getrocknet.
Ausbeute: 6.17 kg (84.3% der Theorie)
Chemische Reinheit laut HPLC: 99.8%

## Patentansprüche

1. Verfahren zur Herstellung der Verbindung der Formel **I** 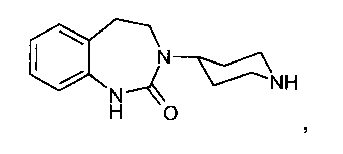 **dadurch gekennzeichnet, dass**
(a) 2-Nitrophenylessigsäure mit 4-Amino-*N*-phenylmethylpiperidin zu 2-Nitro-*N*-[1-(phenylmethyl)-4-piperidinyl]-phenylacetamid der Formel **II** 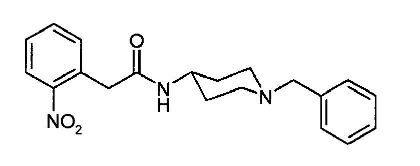 umgesetzt wird;
(b) die Nitrogruppe des erhaltenen 2-Nitro-*N*-[1-(phenylmethyl)-4-piperidinyl]-phenylacetamids der Formel **II** zur Aminogruppe hydriert wird, wodurch die Verbindung der Formel **III** 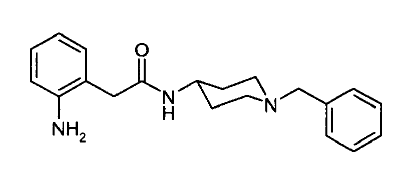 entsteht;
(c) das erhaltene Zwischenprodukt der Formel **III** durch Zugabe eines Esters und in Gegenwart einer Base zu einem Carbamat der allgemeinen Formel **IV** 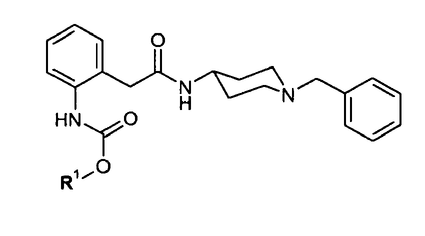 worin **R¹** eine Phenyl-, Benzyl-, Allyl-, 2,2,2-Trichlorethyl- oder eine geradkettige oder verzweigte C₁₋₆-Alkylgruppe bedeutet, umgesetzt wird;
(d) die erhaltene Verbindung der allgemeinen Formel **IV** in einem aprotischen organischen Lösungsmittel gelöst, die Carbonylgruppe durch Zugabe eines Reduktionsmittels in eine Methylengruppe übergeführt und zu 3-[1-(Phenylmethyl)-4-piperidinyl]-2,3,4,5-tetrahydro-1,3-benzodiazepin-2(1*H*)-on der Formel **V** 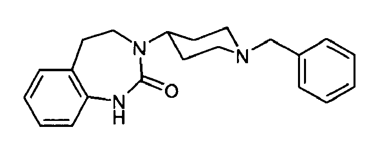 cyclisiert wird und
(e) das in Schritt (d) erhaltene 3-[1-(Phenylmethyl)-4-piperidinyl]-2,3,4,5-tetrahydro-1,3-benzodiazepin-2(1*H*)-on der Formel **V** durch Abspalten der Benzylschutzgruppe in die Verbindung der Formel **I** übergeführt wird.

2. Verfahren gemäß Anspruch 1, **dadurch gekennzeichnet, dass** die in Schritt (a) das 4-Amino-*N*-phenylmethylpiperidin als Lösung in einem polaren aprotischen Lösungsmittel zugegeben und die Reaktion in Gegenwart eines Kondensationsmittels durchgeführt wird.

3. Verfahren gemäß Anspruch 2, **dadurch gekennzeichnet, dass** als polares aprotisches Lösungsmittel Tetrahydrofuran, 2-Methyltetrahydrofuran, Dimethoxyethan, Toluol, Dimethylformamid oder *N*-Methylpyrrolidinon verwendet wird.

4. Verfahren gemäß Anspruch 2, **dadurch gekennzeichnet, dass** in Schritt (a) als Kondensationsmittel Carbonyldiimidazol, Carbonylditriazol, n-Propanphosphonsäureanhydrid, Dicyclohexylcarbodiimid, Thionylchlorid, TBTU O-(Benzotriazol-1-yl)-*N,N,N',N'-*tetramethyluroniumtetrafluoroborat oder 1-Ethyl-3-(3'-dimethylaminopropyl)-carbodiimid verwendet werden.

5. Verfahren gemäß Anspruch 2, **dadurch gekennzeichnet, dass** in Schritt (a) 1.1 bis 1.5 Äquivalente des Kondensationsmittels zugegeben werden.

6. Verfahren gemäß Anspruch 1, **dadurch gekennzeichnet, dass** das in Schritt (a) erhaltene Produkt durch Verdünnen mit Wasser auskristallisiert wird.

7. Verfahren gemäß Anspruch 1, **dadurch gekennzeichnet, dass** die Reaktion in Schritt (b) in einem polaren organischen Lösungsmittel und durch Zugabe eines Katalysators durchgeführt wird.

8. Verfahren gemäß Anspruch 7, **dadurch gekennzeichnet, dass** in Schritt (b) als polares organisches Lösungsmittel Methanol, Ethanol, Isopropanol, Dimethylformamid, *N*-Methylpyrrolidinon, 2-Methyltetrahydrofuran, Tetrahydrofuran, Dioxan oder Dimethoxyethan verwendet wird.

9. Verfahren gemäß Anspruch 7, **dadurch gekennzeichnet, dass** in Schritt (b) als Katalysator Raney-Nickel oder Platindioxid verwendet werden und die Hydrierung bei einer Temperatur von 20 bis 60°C und einem Wasserstoffüberdruck von maximal 3 bar durchgeführt wird.

10. Verfahren gemäß Anspruch 7, **dadurch gekennzeichnet, dass** der Katalysator in Schritt (b) in einer Menge von 2.5 bis 20% zugesetzt wird.

11. Verfahren gemäß Anspruch 1, **dadurch gekennzeichnet, dass** in Schritt (c) 1.0 bis 2.0 Äquivalente eines Esters zugegeben werden.

12. Verfahren gemäß Anspruch 1, **dadurch gekennzeichnet, dass** in Schritt (c) als Ester Pyrokohlensäure-di-*tert.*-butylester oder ein Chlorameisensäureester verwendet werden.

13. Verfahren gemäß Anspruch 1, **dadurch gekennzeichnet, dass** in Schritt (c) 1.0 bis 2.0 Äquivalente der Base zugegeben werden.

14. Verfahren gemäß Anspruch 1, **dadurch gekennzeichnet, dass** in Schritt (c) als Base *N*-Methylmorpholin oder eine wässrige Lösung von Natriumcarbonat, Kaliumcarbonat, Natriumhydroxid, Ammoniak oder Kaliumhydroxid verwendet wird.

15. Verfahren gemäß Anspruch 1, **dadurch gekennzeichnet, dass** in Schritt (d) als aprotisches organisches Lösungsmittel Toluol, 2-Methyltetrahydrofuran, Tetrahydrofuran oder Dimethoxyethan verwendet wird.

16. Verfahren gemäß Anspruch 1, **dadurch gekennzeichnet, dass** in Schritt (d) das Reduktionsmittel in einer Menge von mindestens 2 Äquivalenten zugegeben wird.

17. Verfahren gemäß Anspruch 1, **dadurch gekennzeichnet, dass** in Schritt (d) als Reduktionsmittel Boran, Diisobutylaluminiumhydrid, Lithium- oder Natriumborhydrid verwendet wird.

18. Verfahren gemäß Anspruch 1, **dadurch gekennzeichnet, dass** das in Schritt (d) erhaltene Produkt 3-[1-(Phenylmethyl)-4-piperidinyl]-2,3,4,5-tetrahydro-1,3-benzodiazepin-2(1*H*)-on durch Verdünnen mit Wasser und Alkohol ausgefällt wird.

19. Verfahren gemäß Anspruch 1, **dadurch gekennzeichnet, dass** der Ausgangsstoff in Schritt (e) in einem polaren Lösungsmittel gelöst und nach Zugabe eines Hydrierungsmittels in einem Druckreaktor bei Temperaturen von 40 bis 80°C und einem Wasserstoffüberdruck von maximal 3 bar hydriert wird.

## Claims

1. Process for preparing the compound of formula I 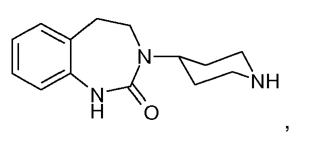 **characterised in that**
(a) 2-nitrophenylacetic acid is reacted with 4-amino-*N*-phenylmethylpiperidine to form 2-nitro-*N*-[1-(phenylmethyl)-4-piperidinyl]-phenylacetamide of formula **II** 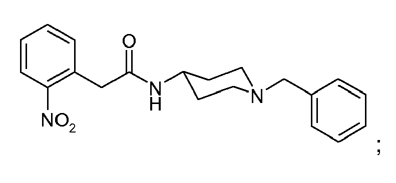
(b) the nitro group of the 2-nitro-*N*-[1-(phenylmethyl)-4-piperidinyl]-phenylacetamide of formula **II** obtained is hydrogenated to form the amino group, thereby producing the compound of formula **III** 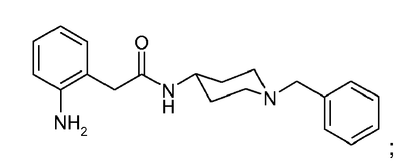
(c) the intermediate product of formula **III** obtained is reacted by the addition of an ester and in the presence of a base to form a carbamate of general formula **IV** 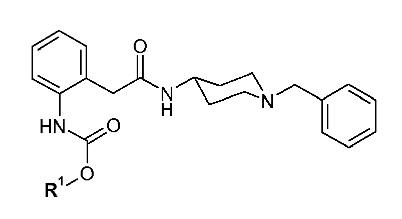 wherein **R¹** denotes a phenyl, benzyl, allyl, 2,2,2-trichloroethyl or a straight-chain or branched C₁₋₆-alkyl group, and
(d) the compound of general formula **IV** obtained is dissolved in an aprotic organic solvent, the carbonyl group is converted into a methylene group by the addition of a reducing agent and cyclised to form 3-[1-(phenylmethyl)-4-piperidinyl]-2,3,4,5-tetrahydro-1,3-benzodiazepin-2(1H)-one of formula **V** 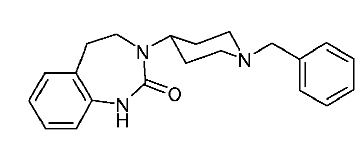 and
(e) the 3-[1-(phenylmethyl)-4-piperidinyl]-2,3,4,5-tetrahydro-1,3-benzodiazepin-2(1 H)-one of formula **V** obtained in step (d) is converted into the compound of formula **I** by cleaving the benzyl protecting group.

2. Process according to claim 1, **characterised in that** in step (a) the 4-amino-*N*-phenylmethylpiperidine is added as a solution in a polar aprotic solvent and the reaction is carried out in the presence of a condensing agent.

3. Process according to claim 2, **characterised in that** tetrahydrofuran, 2-methyltetrahydrofuran, dimethoxyethane, toluene, dimethylformamide or *N-*methylpyrrolidinone is used as the polar aprotic solvent.

4. Process according to claim 2, **characterised in that** in step (a) carbonyldiimidazole, carbonylditriazole, n-propanephosphonic anhydride, dicyclohexylcarbodiimide, thionyl chloride, TBTU O-(benzotriazol-1-yl)-*N,N,N',N'-*tetramethyluronium tetrafluoroborate or 1-ethyl-3-(3'-dimethylaminopropyl)-carbodiimide are used as condensing agents.

5. Process according to claim 2, **characterised in that** in step (a) 1.1 to 1.5 equivalents of the condensing agent are added.

6. Process according to claim 1, **characterised in that** the product obtained in step (a) is crystallised out by diluting with water.

7. Process according to claim 1, **characterised in that** the reaction in step (b) is carried out in a polar organic solvent and by the addition of a catalyst.

8. Process according to claim 7, **characterised in that** in step (b) methanol, ethanol, isopropanol, dimethylformamide, *N*-methylpyrrolidinone, 2-methyltetrahydrofuran, tetrahydrofuran, dioxane or dimethoxyethane is used as the polar organic solvent.

9. Process according to claim 7, **characterised in that** in step (b) Raney nickel or platinum dioxide is used as catalyst and the hydrogenation is carried out at a temperature of 20 to 60°C and under an excess hydrogen pressure of not more than 3 bar.

10. Process according to claim 7, **characterised in that** the catalyst in step (b) is added in an amount of 2.5 to 20%.

11. Process according to claim 1, **characterised in that** in step (c) 1.0 to 2.0 equivalents of an ester are added.

12. Process according to claim 1, **characterised in that** in step (c) di-*tert.*butyl pyrocarbonate or a chloroformic acid ester is used as the ester.

13. Process according to claim 1, **characterised in that** in step (c) 1.0 to 2.0 equivalents of the base are added.

14. Process according to claim 1, **characterised in that** in step (c) *N-*methylmorpholine or an aqueous solution of sodium carbonate, potassium carbonate, sodium hydroxide, ammonia or potassium hydroxide is used as base.

15. Process according to claim 1, **characterised in that** toluene, 2-methyltetrahydrofuran, tetrahydrofuran or dimethoxyethane is used in step (d) as the aprotic organic solvent.

16. Process according to claim 1, **characterised in that** in step (d) the reducing agent is added in an amount of at least 2 equivalents.

17. Process according to claim 1, **characterised in that** in step (d) borane, diisobutylaluminium hydride, lithium or sodium borohydride is used as reducing agent.

18. Process according to claim 1, **characterised in that** the product 3-[1-(phenylmethyl)-4-piperidinyl]-2,3,4,5-tetrahydro-1,3-benzodiazepin-2(1H)-once obtained in step (d) is precipitated by diluting with water and alcohol.

19. Process according to claim 1, **characterised in that** the starting material in step (e) is dissolved in a polar solvent and after the addition of a hydrogenating agent the mixture is hydrogenated in a pressurised reactor at temperatures of 40 to 80°C and under an excess hydrogen pressure of not more than 3 bar.

## Revendications

1. Procédé de production du composé de formule I 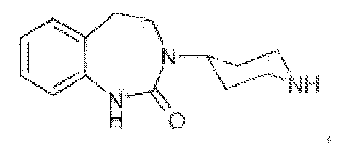 **caractérisé en ce que**
(a) de l'acide 2-nitrophénylacétique est converti avec de la 4-amino-N-phénylméthylpipéridine en 2-nitro-N-[1-(phénylméthyl)-4-pipéridinyl]-phényl-acétamide de formule II 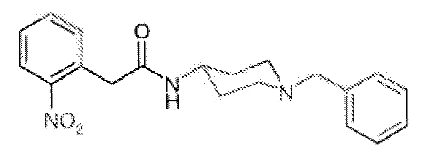
(b) le groupe nitro du 2-nitro-N-[1-(phényl-méthyl)-4-pipéridinylphénylacétamide de formule II obtenu est hydrogéné en groupe amino, par lequel le composé de formule III 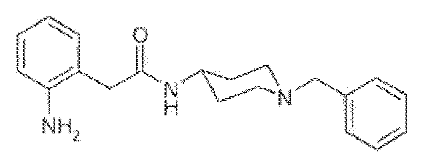 est obtenu,
(c) le produit intermédiaire de formule III obtenu étant converti par addition d'un ester et en présence d'une base, en un carbamate de formule générale IV 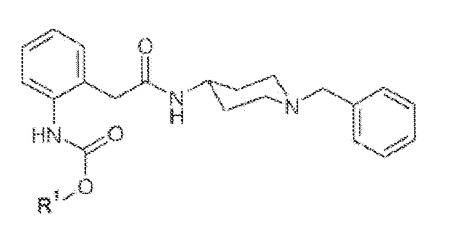 dans laquelle R¹ est une groupe phényle, benzyle, allyle, 2,2,2-trichlorcérthyle ou un groupe alkyle en C₁ à C₆ linéaire ou ramifié,
(d) le composé obtenu de formule générale IV est dissous dans un solvant organique aprotique, le groupe carbonyle est converti par addition d'un agent réducteur en un groupe méthyle et cyclisé en 3- [1- (phénylméthyl) -4-pipéridinyl]-2,3,4,5-tétrahydro-1,3-benzodiazépine-2-(1H)one de formule V 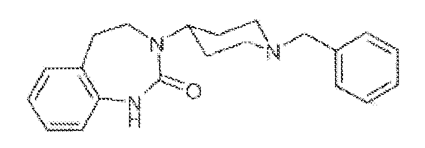 et,
(e) la 3-[1-(phénylméthyl)-4-pipéridinyl]-2,3,4, 5-tétrahydro-1,3-benzodiazépin-2(1H)-one de formule V est convertie par clivage du groupe benzyle protecteur en composé de formule I.

2. Procédé selon la revendication 1, **caractérisé en ce qu'**à l'étape (a), la 4-amino-N-phénylméthylpipéridine en solution est ajoutée dans un solvant aprotique et la réaction est réalisée en présence d'un agent de condensation.

3. Procédé selon la revendication 2 qui utilise comme solvant aprotique polaire, le tétrahydrofurane, le 2-méthyltétrahydrofurane, le diméthoxyéthane, le toluène, le diméthylformamide ou la N-méthylpyrrolidone.

4. Procédé selon la revendication 2, **caractérisé en ce qu'**à l'étape (a), on utilise comme agent de condensation, du carbonyldiimidazol, du carbonylditriazol, de l'anhydride d'acide n-propane-phosphonique, du dicyclohexylcarbodiimide, du chlorure de thionyle, du TBTU O-(benzotriazol-1-yl)-N,N,N',N'-tétraméthyluroniumtétrafluoroborate ou du 1-éthyl-3m(3'-diméthylaminopropyl)-carbodiimide.

5. Procédé selon la revendication 2, **caractérisé en ce qu'**à l'étape (a), on ajoute 1,1 à 1,5 équivalents de l'agent de condensation.

6. Procédé selon la revendication 1, **caractérisé en ce qu'**à l'étape (a), le produit obtenu est recristallisé par dilution avec de l'eau.

7. Procédé selon la revendication 1, **caractérisé en ce que** la réaction est réalisée à l'étape (b) dans un solvant polaire organique et par addition d'un catalyseur.

8. Procédé selon la revendication 7, **caractérisé en ce qu'**à l'étape (b), on utilise comme solvant polaire organique, du méthanol, de l'éthanol, de l'isopropanol, du diméthylformamide, de la N-méthylpyrrolidone, du 2-méthyltétrahydrofurane, du tétrahydrofurane, du dioxane ou du diméthoxyéthane.

9. Procédé selon la revendication 7, **caractérisé en ce qu'** à l'étape (b), on utilise comme catalyseur, du nickel Raney ou du dioxyde de platine et l'hydrogénation est réalisée à une température de 20 à 60°C et à une pression d'hydrogène au maximum de 3 bars.

10. Procédé selon la revendication 7, **caractérisé en ce que** le catalyseur est ajouté à l'étape (b) en une quantité de 2,5 à 20 %,

11. Procédé selon la revendication 1, **caractérisé en ce qu'**à l'étape (c), on ajoute 1,0 à 2,0 équivalents d'un ester,

12. Procédé selon la revendication 1, **caractérisé en ce qu'**à l'étape (c), on utilise comme ester, le di-tert.-butylester d'acide pyrocarboxylique ou un ester d'acide chloroformique.

13. Procédé selon la revendication 1, **caractérisé en ce qu'**à l'étape (c), on ajoute 1,0 à 2,0 équivalents de base.

14. Procédé selon la revendication 1, **caractérisé en ce qu'** à l' étape (c), on utilise comme base de la N-méthylmorpholine ou une solution aqueuse de carbonate de sodium, de carbonate de potassium, d'hydroxyde de sodium, d'ammoniaque ou d'hydroxyde de potassium.

15. Procédé selon la revendication 1, **caractérisé en ce qu'** à l'étape (d), on utilise comme solvant organique aprotique, du toluène, du 2-méthyltétrahydrofurane, du tétrahydrofurane ou du diméthoxyéthane.

16. Procédé selon la revendication 1, **caractérisé en ce qu'**à l'étape (d), l'agent de réduction est ajouté en une quantité d'au moins 2 équivalents,

17. Procédé selon la revendication 1, **caractérisé en ce qu'**à l'étape (d), on utilise comme agent de réduction, du borane, de l'hydrure de diisobutylammonium ou du borohydrure de lithium ou de sodium.

18. Procédé selon la revendication 1, **caractérisé en ce qu'** à l'étape (d), le produit obtenu 3-[1-(phénylméthyl)-4-pipéridinyl]-2,3,4,5-tétrahydro-1,3-benzodiazépine-2(1H)-one est précipité par dilution avec de l'eau et un alcool.

19. Procédé selon la revendication 1, **caractérisé en ce que** le matériau de départ de l'étape (e) est dissous dans un solvant polaire et après addition d'un agent d'hydrogénation, dans un réacteur à pression à des températures entre 40 et 80°C et sous une pression d'hydrogène au maximum de 3 bars.
